(19) Europäisches Patentamt
European Patent Office
Office européen des brevets

(11) Veröffentlichungsnummer: **0 076 413**
**B1**

(12) # EUROPÄISCHE PATENTSCHRIFT

(45) Veröffentlichungstag der Patentschrift: **04.09.85**

(51) Int. Cl.⁴: **C 07 F 7/10**

(21) Anmeldenummer: **82108619.6**

(22) Anmeldetag: **18.09.82**

(54) **Verfahren zur Herstellung von Trimethylsilylcyanid.**

(30) Priorität: **03.10.81 DE 3139456**

(43) Veröffentlichungstag der Anmeldung:
**13.04.83 Patentblatt 83/15**

(45) Bekanntmachung des Hinweises auf die Patenterteilung:
**04.09.85 Patentblatt 85/36**

(84) Benannte Vertragsstaaten:
**CH DE FR GB LI**

(56) Entgegenhaltungen:
**SYNTHESIS**
**M.T. REETZ et al. "An improved synthesis of cyanotrimethylsilane", 1982, Georg Thieme Verlag, Stuttgart, New York, Seite 330**
**Chemical Abstracts Band 91, Nr. 17, 22. Oktober 1979, Columbus, Ohio, USA**
**S. HUENIG et al. "A simple and productive synthesis of trimethylsilyl cyanide", Seite 665, Spalte 1, Abstract Nr. 140901y**
**Chemical Abstracts Band 91, Nr. 17, 22. Oktober 1979, Columbus, Ohio, USA**
**J.K. RASMUSSEN et al. "A simple, safe, and inexpensive preparation of trimethylsilyl cyanide", Seite 665, Spalte 1, Abstract Nr. 140902z**

(73) Patentinhaber: **BAYER AG, Konzernverwaltung RP Patentabteilung, D-5090 Leverkusen 1 Bayerwerk (DE)**

(72) Erfinder: **Reetz, Manfred T., Prof. Dr., Wiesentalweg 14, D-3550 Marburg (DE)**
Erfinder: **Chatziiosifidis, Ioannis, Dr., Butzgrabenweg 28, D-7582 Bühlertal (DE)**

EP 0 076 413 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues, verbessertes Verfahren zur Herstellung von Trimethylsilylcyanid aus Trimethylsilylchlorid. Trimethylsilylcyanid ist ein wertvolles, vielseitig verwendbares Zwischenprodukt in organischen Synthesen.

Die derzeit besten bekannten Methoden zur Herstellung von Trimethylsilylcyanid stammen von S. Hünig et al. (Synthesis 1979, S. 522) und J.K. Rasmussen et al. (Synthesis 1979, S. 523) und gehen beide von Trimethylsilylchlorid und Alkalicyanid aus. Nach Hünig wird Trimethylsilylchlorid mit überschüssigem Natriumcyanid (22% Überschuss) in Gegenwart eines Phasentransferkatalysators und in Gegenwart von N-Methylpyrrolidon als Lösungsmittel 30-36 Stunden bei 90-100°C umgesetzt, wobei man 60-70% an isoliertem Trimethylsilylcyanid erhält. Nach Rasmussen setzt man das Trimethylsilylchlorid mit überschüssigem Kaliumcyanid (250% Überschuss) in N-Methylpyrrolidon 16 Stunden bei Rückflusstemperaturen um und erhält 71% an Trimethylsilylcyanid, isoliert durch Destillation.

Beiden Verfahrensvarianten haftet, abgesehen von den mässigen Ausbeuten, der Nachteil an, dass das Alkalicyanid im Überschuss eingesetzt werden muss und dass relativ lange Reaktionszeiten und höhere Temperaturen benötigt werden. Ausserdem muss bei einer der beiden Verfahrensvarianten, wie angegeben, ein Phasentransferkatalysator zugesetzt werden.

Es wurde nun überraschend gefunden, dass man Trimethylsilylcyanid, $(CH_3)_3Si\text{-}CN$, in hoher Ausbeute und Reinheit durch Umsetzung von Trimethylsilylchlorid mit Alkalicyanid in Gegenwart von N-Methylpyrrolidon erhält, wenn man das Trimethylsilylchlorid unter Ausschluss von Wasser mit der etwa äquimolaren Menge eines Alkalicyanids in Gegenwart von jeweils katalytischen, unterstöchiometrischen Mengen eines Alkali-iodids und von N-Methylpyrrolidon bei einer Temperatur von 15°C bis 25°C umsetzt, wobei die Reaktionszeit bis zu vollständigem Umsatz etwa 12 Stunden beträgt.

Das erfindungsgemässe Verfahren vermeidet die Nachteile der vorbekannten Methoden und stellt somit eine wesentliche Verbesserung dar. So braucht das Alkalicyanid nur in stöchiometrischen Mengen, nicht mehr im Überschuss, eingesetzt zu werden. Das N-Methylpyrrolidon braucht man, ebenso wie das katalytisch wirksame Alkali-iodid, nur in unterstöchiometrischer Menge, nicht aber als Lösungsmittel, einzusetzen. Gleichzeitig erzielt man unter milderen Reaktionsbedingungen — bei nicht erhöhter Temperatur und trotzdem stark verkürzten Reaktionszeiten — wesentlich höhere Ausbeuten (bis zu 90% der Theorie).

Setzt man nach dem erfindungsgemässen Verfahren Trimethylsilylchlorid mit Kaliumcyanid in Gegenwart von (katalytischen Mengen) Kaliumiodid um, so kann der Reaktionsablauf durch das folgende Formelschema wiedergegeben werden:

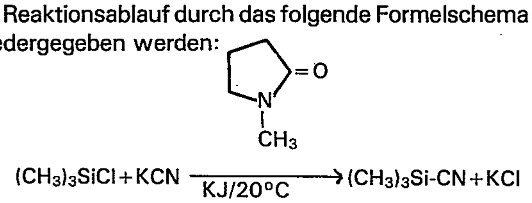


Als Alkalicyanide werden bevorzugt Natriumcyanid und Kaliumcyanid verwendet. Als Alkali-iodid wird vorzugsweise Kaliumiodid der Natriumiodid verwendet.

Bei der Durchführung des erfindungsgemässen Verfahrens setzt man Trimethylsilylchlorid und Alkalicyanid vorzugsweise in exakt stöchiometrischen Mengen um. Alkali-iodid und N-Methylpyrrolidon werden dagegen in unterstöchiometrischen Mengen eingesetzt, das Alkali-iodid im allgemeinen in Mengen von 5-15 Mol-%, vorzugsweise von 8-12 Mol-%, das N-Methylpyrrolidon im allgemeinen in Mengen von 15-25 Mol-%, vorzugsweise von 18-23 Mol-%, jeweils bezogen auf die Einsatzmenge an Trimethylsilylchlorid bzw. Alkalicyanid.

Eine vollständige Umsetzung lässt sich auch ohne Zusatz von N-Methylpyrrolidon erreichen, jedoch erfordert dies erheblich längere Reaktionszeiten (65 Stunden bei 22°C) und Verwendung eines mechanischen Rührers.

Die Umsetzung wird im allgemeinen bei Normaldruck durchgeführt. Eine Inertgas-Atmosphäre ist nicht erforderlich, jedoch muss die Reaktion unter Feuchtigkeitsausschluss durchgeführt werden. Man arbeitet daher in einem gut getrockneten, gegen Luftfeuchtigkeit geschützten Reaktionsgefäss unter Einsatz von sorgfältig getrockneten Reagenzien, soweit erforderlich. Besonders zweckmässig ist es, eine Suspension von Alkalicyanid und Alkali-iodid in Trimethylsilylchlorid vorzulegen, dann das N-Methylpyrrolidon zuzufügen und das Reaktionsgemisch anschliessend bis zur vollständigen Umsetzung (ca. 12 Stunden) bei Raumtemperatur rühren zu lassen.

Das Trimethylsilylcyanid wird am einfachsten durch direkte Destillation aus dem Reaktionsgefäss in reiner Form erhalten. Die Ausbeuten liegen bei 87 bis 90% der Theorie.

Trimethylsilylcyanid ist ein wertvolles, vielseitig verwendbares Zwischenprodukt der organischen Chemie; es dient z.B. zur Umwandlung von Aldehyden und Ketonen in silylierte Cyanhydrine, welche ihrerseits als nützliche, reaktive Zwischenprodukte bekannt sind (vgl. Synthesis 1980, S. 861; ferner Synthesis 1979, S. 522 und 523). Trimethylsilylcyanid kann insbesondere als Ausgangsprodukt zur Herstellung von Acylcyaniden verwendet werden (vgl. z.B. Synthesis 1979, S. 204 und 205), welche ihrerseits zur Synthese von 1,2,4-Triazin-5-onen, die hervorragende herbizide Eigenschaften besitzen, eingesetzt werden können.

So lässt sich Trimethylsilylcyanid beispielsweise durch Umsetzung mit Pivaloylchlorid glatt in Pivaloylcyanid überführen

$$(CH_3)_3C\text{-}COCl + (CH_3)_3SiCN \longrightarrow$$
$$\longrightarrow \underline{(CH_3)_3C\text{-}COCN} + (CH_3)_3SiCl$$

welches nach bekannten Verfahren z.B. in den herbiziden Wirkstoff 3-Methylthio-4-amino-6-tert.-butyl-1,2,4-triazin-5(4H)-on umgewandelt werden kann (vgl. z.B. DE-PS 1 795 784, DE-OS 2 733 180, US-PS 4 175 188, ferner DE-OS 3 003 203, 3 003 541 und 3 009 043).

Durch Umsetzung mit Benzoylchlorid lässt sich

Trimethylsilylcyanid analog in Benzoylcyanid überführen (vgl. Herstellungsbeispiel, das sich ebenfalls nach bekannten Verfahren z.B. zu dem herbiziden Wirkstoff 3-Methyl-4-amino-6-phenyl-1,2,4-triazin-5(4H)-on weiter umsetzen lässt (vgl. z.B. DE-OS 2 224 161, 2 364 474, 2 528 211, 2 708 189).

Die nachfolgenden Beispiele sollen zur weiteren Erläuterung der Erfindung dienen.

*Beispiel 1*

In einem 250-ml-Rundkolben werden zunächst 65,1 g (1 Mol) trocknes Kaliumcyanid eingefüllt; der Kolben wird mit einem (mit Calciumchlorid gefüllten) Trockenrohr verschlossen und kurze Zeit mit einem Bunsenbrenner erhitzt. (Es kann jedoch auch vorher in einer Trockenpistole getrocknet werden.) Nach Abkühlung werden 15,6 g (0,1 Mol) Kaliumiodid und 108,2 g (1 Mol) Trimethylsilylchlorid zugegeben. Diese Suspension wird mit 19,8 g (0,2 Mol) N-Methylpyrrolidon versetzt, und das Reaktionsgemisch wird anschliessend bei 22°C 12 Stunden gerührt (mittels Magnetrührer). Ein H-NMR-Spektrum des Reaktionsgemisches zeigt jetzt vollständigen Umsatz an. Das gebildete Trimethylsilylcyanid wird durch direkte Destillation aus dem Reaktionsgefäss bei einer Siedetemperatur von 112-117°C isoliert; man erhält 87,2 g (≙88% der Theorie) reines Trimethylsilylcyanid.

*Beispiel 2*

Völlig analog vollzieht sich die Reaktion von Natriumcyanid (1 Mol) und Trimethylsilylchlorid (1 Mol) in Gegenwart von Natriumiodid (0,1 Mol) und N-Methylpyrrolidon (0,2 Mol), wobei Ausbeuten von 87-90% der Theorie an Trimethylsilylcyanid erzielt werden. In diesem Fall kann das Natriumiodid auch durch Kaliumiodid (0,1 Mol) ersetzt werden. Die Aufarbeitung erfolgt wie bei Beispiel 1 durch Destillation.

Das H-NMR-Spektrum des destillierten Produkts (Singulett bei $\delta = 0,2$ ppm in $CCl_4$) zeigt keinerlei Verunreinigungen an. Das Produkt ist hydroskopisch und sollte daher vor Luftfeuchtigkeit geschützt werden. Es wird im Eisschrank fest (Schmelzpunkt ca. 15°C).

## Patentansprüche

1. Verfahren zur Herstellung von Trimethylsilylcyanid, $(CH_3)_3Si-CN$, aus Trimethylsilylchlorid und Alkalicyanid in Gegenwart von N-Methylpyrrolidon, dadurch gekennzeichnet, dass man das Trimethylsilylchlorid unter Ausschluss von Wasser mit der etwa äquimolaren Menge eines Alkalicyanids in Gegenwart von jeweils katalytischen, unterstöchiometrischen Mengen eines Alkali-iodids und von N-Methylpyrrolidon bei einer Temperatur von 15°C bis 25°C umsetzt.

2. Verfahren nach Anspruch 1, dadurch gekennzeichnet, dass man das Alkali-iodid in Mengen von 5-15 Mol-%, vorzugsweise von 8-12 Mol-%, bezogen auf die Einsatzmenge von Trimethylsilylchlorid bzw. Alkalicyanid, einsetzt.

3. Verfahren nach Anspruch 1, dadurch gekennzeichnet, dass man das N-Methylpyrrolidon in Mengen von 15-25 Mol-%, vorzugsweise von 18-23 Mol-%, bezogen auf die Einsatzmenge an Trimethylsilylchlorid bzw. Alkalicyanid, einsetzt.

4. Verfahren nach Anspruch 1, dadurch gekennzeichnet, dass man als Alkalicyanid Natriumcyanid oder Kaliumcyanid einsetzt.

5. Verfahren nach Anspruch 1, dadurch gekennzeichnet, dass als Alkali-iodid Kaliumiodid oder Natriumiodid verwendet.

## Claims

1. Process for the preparation of trimethylsilyl cyanide, $(CH_3)_3Si-CN$, from trimethylsilyl chloride and an alkali metal cyanide inthe presence of N-methylpyrrolidone, characterised in that the trimethylsilyl chloride is reacted with an approximately equimolar amount of an alkali metal cyanide in the absence of water and in the presence of catalytic, substoichiometric amounts of both an alkali metal iodide and N-methylpyrrolidone, at a temperature of 15°C to 25°C.

2. Process according to Claim 1, characterised in that the metal iodide is used in amounts of 5-15 mol %, preferably 8-12 mol %, based on the amount of trimethylsilyl chloride or alkali metal cyanide used.

3. Process according to Claim 1, characterised in that the N-methylpyrrolidone is used in amounts of 15-25 mol %, preferably 18 - 23 mol %, based on the amount of trimethylsilyl chloride or alkali metal cyanide used.

4. Process according to Claim 1, characterised in that sodium cyanide or potassium cyanide is employed as the alkali metal cyanide.

5. Process according to Claim 1, characterised in that potassium iodide or sodium iodide is used as the alkali metal iodide.

## Revendications

1. Procédé de production de cyanure de triméthylsilyle $(CH_3)_3Si-CN$ à partir de chlorure de triméthylsilyle et d'un cyanure alcalin en présence de N-méthylpyrrolidone, caractérisé en ce qu'on fait réagir le chlorure de triméthylsilyle en l'absence d'eau avec la quantité à peu près équimolaire d'un cyanure alcalin en présence de quantités inférieures à la stoechiométrie, catalytiques dans chaque cas, d'un iodure alcalin et de N-méthylpyrrolidone à une température de 15°C à 25°C.

2. Procédé suivant la revendication 1, caractérisé en ce qu'on utilise l'iodure alcalin en quantités de 5 à 15 moles %, de préférence de 8 à 12 moles %, par rapport à la quantité utilisée de chlorure de triméthylsilyle ou de cyanure alcalin.

3. Procédé suivant la revendication 1, caractérisé en ce qu'on utilise la N-méthylpyrrolidone en quantités de 15 à 25 moles %, de préférence de 18 à 23 moles %, par rapport à la quantité utilisée de chlorure de triméthylsilyle ou de cyanure alcalin.

4. Procédé suivant la revendication 1, caractérisé en ce qu'on utilise comme cyanure alcalin le cyanure de sodium ou le cyanure de potassium.

5. Procédé suivant la revendication 1, caractérisé en ce qu'on utilise comme iodure alcalin l'iodure de potassium ou l'iodure de sodium.